# EUROPÄISCHE PATENTSCHRIFT

(11) **EP 3 470 401 B1**
(45) Veröffentlichungstag und Bekanntmachung des Hinweises auf die Patenterteilung: **04.11.2020**
(21) Anmeldenummer: 18197224.1
(22) Anmeldetag: 27.09.2018
(51) Int. Cl.: C07D 279/26, C07C 319/14

(54) **VERFAHREN ZUR PHOTOREDOXKATALYTISCHEN ADDITION VON SCHWEFELHEXAFLUORID AN UNGESÄTTIGTE KOHLENSTOFF-KOHLENSTOFF-BINDUNGEN**
METHOD FOR PHOTOREDOX CATALYTIC ADDITION OF SULPHUR HEXAFLUORIDE TO UNSATURATED CARBON-CARBON BONDS
PROCÉDÉ D'ADDITION CATALYTIQUE PHOTOREDOX DE L'HEXAFLUORURE DE SOUFRE À DES LIAISONS CARBONE-CARBONE NON SATURÉES

(30) Priorität: 12.10.2017 DE 102017009501
(43) Veröffentlichungstag der Anmeldung: 17.04.2019
(73) Patentinhaber: Karlsruher Institut für Technologie, 76131 Karlsruhe (DE)
(72) Erfinder: Wagenknecht, Hans-Achim, 75045 Walzbachtal (DE); Rombach, David, 76199 Karlsruhe (DE)
(74) Vertreter: Müller-Boré & Partner Patentanwälte PartG mbB

(56) Entgegenhaltungen:
- GRELBIG, T. ET AL.: "Additionen von SF5Cl und TeF5Cl an C=C Doppelbindungen", ZEITSCHRIFT FÜR ANORGANISCHE UND ALLGEMEINE CHEMIE, Bd. 544, 1987, Seiten 74-80, XP002786309,
- ROMBACH, D.; WAGENKNECHT, H.-A.: "Photoredox Catalytic Activation of Sulfur Hexafluoride for Pentafluorosulfanylation of alpha-Methyl and alpha-Phenyl Styrene", CHEMCATCHEM, Bd. 10, Nr. 14, 4. April 2018 (2018-04-04) , Seiten 2955-2961, XP002786310,

## Beschreibung

Die vorliegende Erfindung betrifft ein Verfahren zur photoredoxkatalytischen Addition von Schwefelhexafluorid (SF₆) an ein Edukt mit ungesättigter Kohlenstoff-Kohlenstoff-Bindung.

Eine der größten Herausforderungen an die Chemie im 21. Jahrhundert ist die verstärkte Nutzung des Lichtes, beispielsweise das preiswert von LEDs erzeugte Licht oder am besten das der Sonne. Auch die organische Synthesechemie stellt sich dieser Herausforderung in zunehmendem Maße. In klassischen photochemischen Reaktionen wird die Lichtenergie direkt in das Substrat eingestrahlt oder von einem Sensibilisator absorbiert und übertragen (N. Hoffmann, Chem. Rev. 2008, 108, 1052-1103; A. G. Griesbeck, J. Mattay, a) Photochemical Key Steps in Organic Synthesis, VCH, Weinheim, 1994; b) Synthetic Organic Photochemistry, Marcel Dekker, New York, 2005).

Moderner geprägt ist der Begriff der chemischen Photokatalyse, wobei dessen Gebrauch nicht einheitlich ist. Der Begriff wird immer dann für lichtinduzierte Reaktionen eingesetzt, wenn ein Photokatalysator und nicht das Edukt durch Licht angeregt wird und die Energie oder ein Elektron auf das Edukt (Substrat) oder den Photokatalysator übertragen werden. Grundsätzlich gilt, wie bei jedem Katalysator, dass auch der Photokatalysator nach Entstehung des Produkts unverändert freigesetzt wird und zurückgewonnen werden kann. Ein chemischer Photokatalysator ist ein Stoff (eine Verbindung), der den physikalischen Lichtanregungsprozess mit einer chemischen Folgereaktion zeitlich, räumlich und energetisch koppelt, ggf. unter Nutzung von Zwischenschritten, z. B. bei der Stabilisierung einer Ladungstrennung (M. Fagnoni, D. Dondi, D. Ravelli, A. Albini, Chem. Rev. 2007, 107, 2725-2756).

Die Photokatalyse, insbesondere die Photoredoxkatalyse mit sichtbarem Licht hat in der organischen Synthesechemie in den letzten fünf Jahren stetig zunehmend Anwendungen gefunden. Beispielhaft seien hier einige Übersichtsartikel der Autoren A. Albini (D. Ravelli, D. Dondi, M. Fagnoni, A. Albini, Chem. Soc. Rev. 2009, 38, 1999-2011; D. Ravelli, M. Fagnoni, A. Albini, Chem. Soc. Rev. 2013, 42, 97-113), B. König (D. P. Hari, B. König, Angew. Chem. Int. Ed. 2013, 52, 4734-4743), D. W. C. MacMillan (C. K. Prier, D. A. Rankic, D. W. C. MacMillan, Chem. Rev. 2013, 113, 5322-5363), C. R. J. Stephenson (J. M. R. Naryanam, C. R. J. Stephenson, Chem. Soc. Rev. 2011, 40, 102-113; J. W. Tucker, C. R. J. Stephenson, J. Org. Chem. 2012, 77, 1617-1622), Y. Xi (Y. Xi, H. Yi, A. Lei, Org. Biomol. Chem. 2013, 11, 2387-2403) und T. P. Yoon (T. P. Yoon, M. A. Ischay, J. Du, Nature Chem. 2010, 2, 510-532) erwähnt. Das Repertoire photokatalytischer organischer Reaktion ist unterdessen extrem breit, beispielhaft seien hier [2+2]-Cycloadditionen, Arylierungen, Cyclisierungen und photoredoxorganokatalytische C-C-Verknüpfungen mit hoher Stereoselektivität genannt.

Üblicherweise umfasst ein Photoredoxkatalysator einen redoxaktiven Chromophor. Bei der Photoredoxkatalyse findet ein Elektronentransfer statt, welcher zur Bildung von Radikalionen führt. Dadurch wird eine organisch-chemische Reaktion initiiert. Der Redoxprozess wird innerhalb des Katalysezyklus durch Rückübertragung des Elektrons (*back electron transfer* (BET)) zum oder vom redoxaktiven Chromophor wieder geschlossen, wodurch der Photoredoxkatalysator am Ende unverändert freigesetzt wird.

Einer der ersten "Meilensteine" in der Geschichte der Darstellung trifluormethylierter Verbindungen ist die Umsetzung von Trichlortoluol mit Antimonpentafluorid durch Swarts im Jahre 1892 (F. Swarts Acad. Roy. Belg. 1892, 3, 24, 474). Sie legte den Grundstein für die Entwicklung der modernen organischen Fluorchemie. Der pharmakologische Wert fluorierter funktioneller Gruppen wurde am Profil der Trifluormethylgruppe bereits 1927 von F. Lehmann erkannt (F. Lehmann, Pathol. Pharmakol. 1928, 130, 250-255). Während der zurückliegenden 50 Jahre fokussierten sich die Bemühungen im Forschungsfeld der organischen Fluorchemie im Wesentlichen auf die Entwicklung effizienter Methoden zur Darstellung fluorierter sowie trifluormethylierter Verbindungen sowie deren Derivate (beispielsweise Trifluormethylether, Trifluormethylthioether sowie Difluormethylderivate). Während die Chemie der Trifluormethylverbindungen seit den späten 1980er Jahren ein exponentiell ansteigendes Forschungsinteresse auf sich zog, das zur Entwicklung einer Vielzahl von ausgereiften Methoden führte, wurde bis heute nicht über die Entwicklung einer vergleichbar ausgereiften Funktionalisierungsmethode zur Einführung der chalkogenhomologen Pentafluorsulfanylgruppe (R-SFs bzw. · SF₅) berichtet. Die bisher extrem schwierige Zugänglichkeit von Pentafluorsulfanylverbindungen, gepaart mit herausragenden pharmakologischen Eigenschaften, führte dazu, dass die Pentafluorsulfanylgruppe in verschiedenen Arbeiten als "Substituent der Zukunft" betrachtet wird (vgl. X. Mi, J. Chen, L. Xu Eur. J. Org. Chem. 2015, 7, 1415-1418; Scriven, Heterocyclic Chemistry in the 21st Century: A Tribute to Alan Katritzky, Elsevier 2016, 40; und H. R. A. Golf, H.-U. Reissig, Arno W. J. Org. Chem. 2015, 80, 5133-5143).

In verschiedenen Arbeiten wurde gezeigt, dass die Substitution der Trifluormethylgruppe durch die SF₅-Gruppe in vielen Fällen eine Verbesserung des pharmakologischen Wirkprofils erwirkt, was an verschiedenen Eigenschaften des Substituenten nachvollzogen werden kann (vgl. P. R. Savoie, J. T. Welch Chem. Rev. 2015, 115, 1130-1190). Einerseits weist die SF₅-Gruppe aufgrund ihrer quadratisch-pyramidalen Geometrie eine verminderte Rotationsbarriere auf und ist aus diesem Grunde in der Lage, mit hoher Bindungskonstante mit biologischen Strukturen zu wechselwirken (vgl. P. R. Savoie, J. T. Welch Chem. Rev. 2015, 115, 1130-1190). Weiterhin vermindert die hohe hydrolytische Beständigkeit der funktionellen Gruppe SF₅ unerwünschte kovalente Enzyminhibition, wie sie im Falle von Trifluormethylverbindungen insbesondere in alkalischem Milieu beobachtet wird. Weiterhin zeichnen sich Verbindungen mit SF₅-Gruppe durch einen stärkeren elektronenziehenden Charakter, der sich auf den pKs-Wert umliegender Protonen sowie die Lipophilie der Verbindungen auswirkt, aus. Somit weisen zahlreche Verbindungen mit SF₅-Gruppe eine großes biologisches, medizinisch-chemisches und pharmokologisches Potential auf.

Die derzeit etablierten Methoden zur Einführung des Pentafluorsulfanylsubstituenten basieren auf der Verwendung hochtoxischer, gasförmiger Reagenzien in frühen Synthesestufen. Hierbei werden die extrem toxischen Verbindungen SF₅Cl und SFsBr sowie S₂F₁₀ verwendet, die auf dem europäischen Markt teils nicht bzw. nur schlecht verfügbar sind und außerordentlich besonderen Sicherheitsbestimmungen unterliegen. Diese Restriktionen führen zu einer Unterentwicklung der Methoden zur synthetischen Einführung der SF₅-Gruppe.

McTeague und Jamison befassen sich mit der photochemischen Aktivierung von Schwefelhexafluorid und der synthetischen Nutzung von SF₆ und beschreiben, wie SF₆ zur Desoxyfluorierung von aktivierten Alkoholen unter stark reduktiven Bedingungen verwendet werden kann, wobei eine Mehrelektronenreduktion des Schwefelhexafluorids stattfindet (T. A. McTeague, T. F. Jamison, Angew. Chem. Int. Ed. 2016, 55, 15072).

Weitere Arbeiten beschäftigen sich mit metallkatalysiertem vollständigen Abbau von Schwefelhexafluorid ohne synthetische Nutzung der Reduktionsprodukte. Zudem wurde die Aktivierung von SF₆ mithilfe von Metallocenen (bspw. Vanadocen) beschrieben (vgl. L. Zámostná, T. Braun, Angew. Chem. Int. Ed. 2015, 54, 10652 - 10656; und B. G. Harvey, A. M. Arif, A. Glöckner, R. D. Ernst, Organometallics 2007, 26 (11), 2872-2879).

Ein Verfahren zur photochemischen Einführung einer Pentafluorsulfanylgruppe unter Verwendung des ungiftigen Schwefelhexafluorids in organische Substrate (Edukte) ist bislang nicht bekannt.

Somit liegt der vorliegenden Erfindung die Aufgabe zugrunde, ein Verfahren zur photoredoxkatalytischen Addition von Schwefelhexafluorid an ein Edukt mit ungesättigter Kohlenstoff-Kohlenstoff-Bindung bereitzustellen, welches effizient, nachhaltig und umweltfreundlich sein soll.

Die vorstehende technische Aufgabe wird durch die in den Ansprüchen gekennzeichneten Ausführungsformen gelöst.

In einem ersten Aspekt betriff die vorliegende Erfindung ein Verfahren zur photoredoxkatalytischen Addition von Schwefelhexafluorid an ein Edukt mit ungesättigter Kohlenstoff-Kohlenstoff-Bindung, umfassend die Schritte
(A) Bereitstellen eines Reaktionsgemisches, welches das Edukt, einen Photoredoxkatalysator sowie Schwefelhexafluorid, umfasst; und
(B) Bestrahlen des Reaktionsgemisches mit elektromagnetischer Strahlung, welche UV-Licht und/oder Licht aus dem sichtbaren Wellenlängenbereich ist, aus einer High-Power-LED als Lichtquelle,
wobei der Photoredoxkatalysator ein Photoredoxkatalysator mit einer N-Phe¬nyl¬phenothiazin-Grundstruktur ist und die ungesättigte Kohlenstoff-Kohlenstoff-Bindung eine Doppelbindung ist.

Durch das erfindungsgemäße Verfahren kann Schwefelhexafluorid an eine ungesättigte Kohlenstoff-Kohlenstoff-Bindung addiert werden. "Addition von Schwefelhexafluorid an eine ungesättigte Kohlenstoff-Kohlenstoff-Bindung" bedeutet erfindungsgemäß, dass an ein Kohlenstoffatom der ungesättigten Kohlenstoff-Kohlenstoff-Bindung durch das erfindungsgemäße Verfahren eine Pentafluorsulfanylgruppe (SFs-Gruppe) und an ein anderes Kohlenstoffatom der ungesättigten Kohlenstoff-Kohlenstoff-Bindung ein Fluoratom gebunden wird. Durch das erfindungsgemäße Verfahren kann somit ein pentafluorsulfanyliertes Produkt erhalten werden.

Ohne durch eine bestimmte Theorie eingeschränkt zu sein, wird für das erfindungsgemäße Verfahren folgender Mechanismus angenommen: Durch das Bestrahlen mit elektromagnetischer Strahlung und Anregung durch ein Photon findet ein Einelektronentransfer von dem Photoredoxkatalysator auf SF₆ unter Bildung eines Schwefelhexafluoridradikalanions (·SF₆⁻) (Reduktion) und Oxidation des Photoredoxkatalysators statt. Das Schwefelhexafluoridradikalanion zerfällt in ein Fluoridanion (F⁻) und ein Pentafluorsulfanylradikal (·SF₅). Das entstandene Radikalkation des Photoredoxkatalysators (Photredoxkatalysator-Radikalkation) wird durch ein weiteres Photon angeregt und ist nun unter Rückelektronentransfer und Rückbildung des Photoredoxkatalysators in der Lage, das Substrat zum Radikalkation zu oxidieren. Dieses Radikalkation wird durch das Pentafluorsulfanylradikal unter Erzeugung eines (gegebenenfalls stabilisierten) Carbokations abgefangen. Das Carbokation addiert nun das bereitgestellte Fluoridanion unter Bildung des gewünschten Additionsprodukts (pentafluorsulfanyliertes Produkt).

Im Allgemeinen kann eine ungesättigte Kohlenstoff-Kohlenstoff-Bindung sowohl eine Doppel- als auch eine Dreifachbindung sein, wobei im erfindungsgemäßen Verfahren die Kohlenstoff-Kohlenstoff-Bindung eine Doppelbindung ist. Mit dem erfindungsgemäßen Verfahren kann sowohl an eine alleinstehende Doppelbindung Schwefelhexafluorid addiert werden, als auch an eine Doppelbindung, welche Teil eines konjugierten π-Elektronensystems, beispielsweise eines aromatischen Systems, ist. Vorzugsweise erfolgt die Addition von Schwefelhexafluorid an eine Kohlenstoff-Kohlenstoff-Doppelbindung, welche Teil eines konjugierten π-Elektronensystems, insbesondere eines aromatischen Systems, ist.

Das in dem erfindungsgemäßen Verfahren verwendete Edukt unterliegt keiner besonderen Einschränkung, solange es dem Erfordernis genügt, dass es eine ungesättigte Kohlenstoff-Kohlenstoff-Doppelbindung aufweist. Das Edukt kann beispielsweise eine metallorganische Verbindung oder eine organische Verbindung sein, wobei eine organische Verbindung bevorzugt ist. Besonders bevorzugt besteht das Edukt aus Atomen, ausgewählt aus der Gruppe, bestehend aus Wasserstoff, Kohlenstoff, Stickstoff, Sauerstoff, Fluor, Brom, Chlor und Schwefel.

Vorzugsweise weist das Edukt ein Reduktionspotential auf, welches kleiner als das Reduktionspotential des Photoredoxkatalysator-Radikalkations ist.

In Bezug auf das Molekülgewicht des Edukts besteht keine besondere Einschränkung. Vorzugsweise beträgt das zahlengemittelte Molekülgewicht des Edukts nicht mehr als 1000 g/mol, besonders bevorzugt nicht mehr als 500 g/mol.

In Schritt (A) des erfindungsgemäßen Verfahrens wird ein Reaktionsgemisch bereitgestellt, welches das Edukt, einen Photoredoxkatalysator sowie Schwefelhexafluorid umfasst.

Gemäß der vorliegenden Erfindung unterliegt das Reaktionsgemisch keiner besonderen Einschränkung. Das Reaktionsgemisch kann in einem einheitlichen (in einem einzigen) Aggregatzustand vorliegen. Das heißt, das Reaktionsgemisch kann fest, flüssig oder gasförmig sein, wobei ein flüssiger oder gasförmiger Zustand bevorzugt ist. Es ist allerdings auch möglich, dass das Reaktionsgemisch mehrere Aggregatszustände aufweist. Beispielsweise können das Edukt und der Photoredoxkatalysator in einem anderen Aggregatszustand vorliegen als das Schwefelhexafluorid.

Gemäß einer bevorzugten Ausführungsform der vorliegenden Erfindung umfasst das Reaktionsgemisch neben dem Edukt, dem Photoredoxkatalysator und dem Schwefelhexafluorid zusätzlich ein Lösungsmittel oder ein Lösungsmittelgemisch. Ein geeignetes Lösungsmittel ist beispielsweise Acetonitril. Erfindungsgemäß können deuterierte, teildeuterierte und nicht-deuterierte Lösungsmittel bzw. Lösungsmittelgemische verwendet werden. Das Reaktionsgemisch enthält vorzugsweise 950 bis 3800 Mol%, besonders bevorzugt 1800 bis 2000 Mol% des Lösungsmittel(gemische)s.

Die hierin angegebenen relativen Stoffmengenwerte in "Mol%" beziehen sich auf die Stoffmenge des Edukts im Reaktionsgemisch unmittelbar vor Schritt (B). Konkret entspricht eine absolute Stoffmenge bei einer relativen Stoffmenge von "100 Mol%" der (absoluten) Stoffmenge des Edukts, und bei einer relativen Stoffmenge von "200 Mol%" liegt die zweifache Stoffmenge des Edukts vor.

Gemäß einer besonders bevorzugten Ausführungsform der vorliegenden Erfindung umfasst das Reaktionsgemisch eine Lösung, welche das Edukt, den Photoredoxkatalysator sowie Schwefelhexafluorid, enthält. Insbesondere kann das Reaktionsgemisch aus einer Flüssigphase, gebildet durch eine Lösung, enthaltend das Edukt, den Photoredoxkatalysator sowie Schwefelhexafluorid, und einer Gasphase, umfassend Schwefelhexafluorid, bestehen. Die Gasphase kann neben Schwefelhexafluorid weitere Bestandteile, beispielsweise ein Inertgas wie Argon oder Stickstoff, enthalten. Vorzugsweise besteht die Gasphase aus Schwefelhexafluorid.

Gemäß der vorliegenden Erfindung ist bevorzugt, dass das Reaktionsgemisch nicht mehr als 50 ppm, vorzugsweise nicht mehr als 5 ppm Sauerstoff enthält. Durch eine niedrige Sauerstoffkonzentration im Reaktionsgemisch kann die Ausbeute an pentafluorsulfanyliertem Produkt erhöht werden.

Gemäß der vorliegenden Erfindung ist bevorzugt, dass das Reaktionsgemisch nicht mehr als 50 ppm, vorzugsweise nicht mehr als 4 ppm Wasser enthält. Durch eine niedrige Wasserkonzentration im Reaktionsgemisch kann die Ausbeute an pentafluorsulfanyliertem Produkt erhöht werden.

Die hierin angegebenen Werte in "ppm" (*parts per million*) beziehen sich auf die Stoffmenge des Edukts im Reaktionsgemisch unmittelbar vor Schritt (B).

Des Weiteren ist bevorzugt, dass das Reaktionsgemisch weitgehend frei von metallorganischen Verbindungen, wie Metallocenen, beispielsweise Vanadocen oder Ferrocen, ist. Zudem ist bevorzugt, dass das Reaktionsgemisch weitgehend frei von SFsCl, SFsBr und S₂F₁₀ ist, da, wie bereits vorstehend erwähnt, diese Verbindungen toxisch sind. Allerdings kann das erfindungsgemäße Verfahren in Gegenwart der vorstehend aufgeführten Verbindungen durchgeführt werden, da diese unter UV-Bestrahlung photolysieren. Erfindungsgemäß bedeutet "weitgehend frei von einer Verbindung", dass höchstens 50 ppm, vorzugsweise höchstens 5 ppm der Verbindung in dem Reaktionsgemisch enthalten sind.

Gemäß einer bevorzugten Ausführungsform der vorliegenden Erfindung enthält das Reaktionsgemisch weiter einen Radikalstabilisator. Geeignet sind insbesondere Radikalstabilisatoren zur Durchführung von thermischen Reaktionen. Derartige Radikalstabilisatoren sind dem Fachmann bekannt (siehe beispielsweise X. Qi, L. Zhu, R. Bai, Y. Lan, Scientific Reports 7, 43579). Erfindungsgemäß ist insbesondere Kupferacetylacetonat (Cu(acac)₂) als Radikalstabilisator geeignet. Die relative Stoffmenge des Radikalstabilisators im Reaktionsgemisch, bezogen auf die Stoffmenge des Edukts, beträgt vorzugsweise 0,1 bis 100 Mol%, besonders bevorzugt 10 bis 50 Mol%. Ohne durch eine bestimmte Theorie beschränkt zu sein, scheint in Schritt (B) ein Pentafluorsulfanylradikal gebildet zu werden, dessen Lebensdauer anscheinend durch Gegenwart eines Radikalstabilisators erhöht werden kann, wodurch das pentafluorsulfanylierte Produkt mit höheren Ausbeuten und deutlich höheren Selektivitäten erhalten werden kann.

Der in dem erfindungsgemäßen Verfahren verwendete Photoredoxkatalysator mit einer N-Phenylphenothiazin-Grundstruktur unterliegt keiner besonderen Einschränkung. Geeignete Photoredoxkatalysatoren sind dem Fachmann bekannt.

Die relative Stoffmenge des Photoredoxkatalysators im Reaktionsgemisch beträgt vorzugsweise 0,01 bis 100 Mol%, besonders bevorzugt 1 bis 20 Mol%, bezogen auf die Stoffmenge des Edukts.

Erfindungsgemäß ist der Photoredoxkatalysator ein Photoredoxkatalysator mit einer N-Phenylphenothiazin-Grundstruktur. Erfindungsgemäß weist der Photoredoxkatalysator dann die Grundstruktur einer bestimmten Verbindung auf, wenn der Photoredoxkatalysator die Verbindung selbst oder ein Derivat davon ist, wobei in dem Derivat ein oder mehrere Wasserstoffatome der Verbindung durch Substituenten ersetzt sind. Diese Substituenten können beispielsweise die gleichen sein, wie nachstehend für die Reste R₁ bis R₁₃ definiert.

Im Allgemeinen können als Photoredoxkatalysator alle organischen und metallorganischen Verbindungen verwendet werden, die eine ähnliche elektronische Struktur wie N-Phenylphenothiazin aufweisen. Eine ähnliche elektronische Struktur liegt beispielsweise dann vor, wenn der Photoredoxkatalysator ein mit dem Reduktionspotential von N-Phenylphenothiazin vergleichbares Reduktionspotential und/oder mit den Absorptionseingenschaften von N-Phenylphenothiazin vergleichbare Absorptionseigenschaften aufweist. Ein vergleichbares Reduktionspotential liegt insbesondere dann vor, wenn der Photoredoxkatalysator das 0,8- bis 1,2-, vorzugsweise das 0,9- bis 1,1-fache Reduktionspotential von N-Phenylphenothiazin aufweist. Vergleichbare Absorptionseigenschaften liegen insbesondere dann vor, wenn die Gesamtabsorption des Photokatalysators im Bereich von 315 bis 500 nm das 0,5- bis 1,5-, vorzugsweise das 0,9- bis 1,1-fache der Gesamtabsorption von N-Phenylphenothiazin im Bereich von 315 bis 500 nm aufweist.

In einer bevorzugten Ausführungsform des erfindungsgemäßen Verfahrens umfasst oder ist der Photoredoxkatalysator eine Verbindung der folgenden Formel (1) 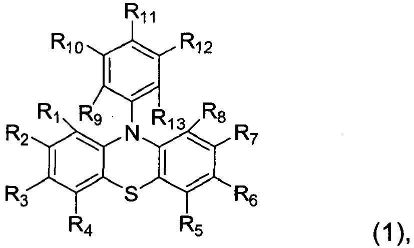 wobei die Reste R₁ bis R₁₃ unabhängig voneinander ein Wasserstoffatom, ein Deuteriumatom, ein Halogenatom, eine Cyanogruppe, eine Nitrogruppe, eine Trifluormethylgruppe, ein Fluoratom, eine substituierte oder unsubstituierte Kohlenwasserstoffgruppe, welche gegebenenfalls ein oder mehrere Heteroatome enthält, oder einen substituierten oder unsubstituierten aromatischen Rest darstellen.

Die Reste R₁ bis R₁₃ sind unabhängig voneinander vorzugsweise ein Wasserstoffatom, ein Deuteriumatom, eine substituierte oder unsubstituierte Kohlenwasserstoffgruppe, welche gegebenenfalls ein oder mehrere Heteroatome enthält, oder ein substituierter oder unsubstituierter aromatischer Rest.

Erfindungsgemäß ist besonders bevorzugt, dass der Photoredoxkatalysator N-Phenylphenothiazin umfasst bzw. N-Phenylphenothiazin ist. In diesem Fall ist jedes von R₁ bis R₁₃ der vorstehenden Formel (1) ein Wasserstoffatom.

Gemäß einer bevorzugten Ausführungsform der vorliegenden Erfindung umfasst bzw. ist das Edukt eine Verbindung der folgenden Formel (I) 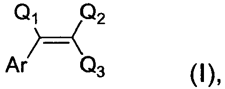 wobei die Reste Q₁ bis Q₃ unabhängig voneinander ein Wasserstoffatom, ein Deuteriumatom, eine Cyanogruppe, eine Trifluormethylgruppe, eine substituierte oder unsubstituierte Kohlenwasserstoffgruppe, welche gegebenenfalls ein oder mehrere Heteroatome enthält, oder einen substituierten oder unsubstituierten aromatischen Rest darstellen und Ar ein aromatischer Rest ist.

Vorzugsweise ist der aromatische Rest Ar eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe, wobei eine substituierte oder unsubstituierte aromatische Kohlenwasserstoffgruppe bevorzugt ist. Gemäß einer besonders bevorzugten Ausführungsform sind der aromatische Rest Ar und der Rest Q₁ unabhängig voneinander jeweils eine substituierte oder unsubstituierte Phenylgruppe, wobei am meisten bevorzugt ist, dass jedes von Ar und Q₁ eine unsubstituierte Phenylgruppe ist.

"Unsubstituiert" bedeutet erfindungsgemäß, dass kein Wasserstoffatom durch einen von einem Wasserstoffatom verschiedenen Substituenten ersetzt ist.

Die substituierte oder unsubstituierte, gegebenenfalls ein oder mehrere Heteroatome enthaltende Kohlenwasserstoffgruppe der vorstehenden Formel (1) bzw. der vorherstehenden Formel (I) kann beispielsweise ein geradkettiges oder verzweigtes Alkyl mit 1 bis 6 Kohlenstoffatomen, welche einen Substituenten aufweisen können, Cycloalkyl mit 5 bis 10 Kohlenstoffatomen, welche einen Substituenten aufweisen können, geradkettiges oder verzweigtes Alkenyl mit 2 bis 6 Kohlenstoffatomen, welche einen Substituenten aufweisen können, geradkettiges oder verzweigtes Alkyloxy mit 1 bis 6 Kohlenstoffatomen, welche einen Substituenten aufweisen können, oder Cycloalkyloxy mit 5 bis 10 Kohlenstoffatomen, welche einen Substituenten aufweisen können, sein. Sofern ein Heteroatom vorhanden ist, ist dieses vorzugsweise aus der Gruppe, bestehend aus Sauerstoff, Stickstoff und Schwefel, ausgewählt.

Gemäß einer bevorzugten Ausführungsform der vorliegenden Erfindung ist das Edukt eine Verbindung mit einer Styrol-Grundstruktur. Für Edukte mit Styrol-Grundstruktur kann durch das erfindungsgemäße Verfahren das pentafluorsulfanylierte Produkt mit besonders hoher Ausbeute erhalten werden.

Gemäß einer bevorzugten Ausführungsform der vorliegenden Erfindung umfasst bzw. ist das Edukt eine Verbindung der folgenden Formel (II) 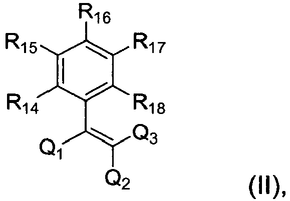 wobei für die Reste Q₁, Q₂ und Q₃ von Formel (II) das gleiche wie für die Reste Q₁, Q₂ und Q₃ von Formel (I) gilt und für die Reste R₁₄ bis R₁₈ das gleiche wie für die Reste R₁ bis R₁₃ von Formel (1) gilt. Vorzugsweise ist jedes von R₁₄ bis R₁₈ ein Wasserstoffatom. Zudem ist bevorzugt, dass Q₁ eine unsubstituierte Phenylgruppe ist.

In Schritt (B) des erfindungsgemäßen Verfahrens wird das Reaktionsgemisch mit elektromagnetischer Strahlung bestrahlt. Erfindungsgemäß ist die elektromagnetische Strahlung UV-Licht (d. h. elektromagnetische Strahlung mit einer Wellenlänge von 100 nm bis kleiner als 400 nm) und/oder Licht aus dem sichtbaren Wellenlängenbereich (d. h. elektromagnetische Strahlung mit einer Wellenlänge von 400 bis 800 nm).

Die in Schritt (B) verwendete elektromagnetische Strahlung kann sowohl monochromatisches Licht als auch Licht (elektromagnetische Strahlung), welches eine Überlagerung von elektromagnetischer Strahlung verschiedener Wellenlängen darstellt, sein. Vorzugsweise wird monochromatisches Licht oder schmalbandiges Licht verwendet. "Schmalbandig" bedeutet in diesem Zusammenhang, dass die elektromagnetische Strahlung einen Wellenlängenbereich von höchstens 200 nm, bevorzugt höchstens 150 nm, insbesondere bevorzugt höchstens 100 nm abdeckt. Gemäß einer bevorzugten Ausführungsform des erfindungsgemäßen Verfahrens ist die elektromagnetische Strahlung Licht aus dem sichtbaren Wellenlängenbereich mit einer Wellenlänge von 400 bis 600 nm und/oder UV-A Licht. Erfindungsgemäß weist UV-A-Licht eine Wellenlänge von mindestens 315 nm bis weniger als 400 nm auf. Das heißt, es ist bevorzugt, dass die Wellenlänge der elektromagnetischen Strahlung 315 bis 500 nm beträgt. Insbesondere ist bevorzugt, dass die Wellenlänge der in Schritt (B) verwendeten elektromagnetischen Strahlung 350 bis 400 nm beträgt. Bei Verwendung von elektromagnetischer Strahlung aus vorstehenden Wellenlängenbereichen können in dem erfindungsgemäßen Verfahren besonders hohe Ausbeuten an pentafluorsulfanyliertem Produkt erzielt werden. Insbesondere eine Kombination von Licht im Wellenlängenbereich von 400 bis 600 nm mit UV-A-Licht führt zu hohen Produktausbeuten.

Bevorzugte Lichtleistungen liegen zwischen 500 und 2000 mW. Als Lichtquelle wird erfindungsgemäß eine High-Power-LED verwendet. Im Allgemeinen können konventionelle Energiesparlampen sowie Hg-Lampen, gegebenenfalls unter Verwendung von Filtern, verwendet werden.

Die vorliegende Erfindung wird anhand der folgenden, nicht einschränkenden Beispiele näher erläutert.

### Beispiele

### Addition von Schwefelhexafluorid an 1,1-Diphenylethen

In einem Schlenkrohr wurden 18,0 mg (17,6 µL, 0,100 mmol, 1,00 eq.) 1,1-Diphenylethen unter Argonatmosphäre in 1 mL absolutem MeCN gelöst. Anschließend wurden 1,4 mg (0,005 mmol, 5 Mol%) N-Phenylphenothiazin sowie 2,6 mg (0,010 mmol, 10 Mol%) Cu(acac)₂ zugegeben. Die Reaktionsmischung wurde drei Male unter Verwendung der gewöhnlichen Freeze-Pump-Thaw-Methode von Sauerstoff befreit. Die Freeze-Pump-Thaw-Methode ist dem Fachmann bekannt. Anschließend wurde die Atmosphäre gegen Schwefelhexafluorid (1 bis 5 bar) getauscht. Das Reaktionsgemisch wurde bei 20°C für 21 h bei 365 nm und 525 nm unter Verwendung einer High-Power-LED belichtet/bestrahlt. Die Produktausbeute wurde nach Zugabe von deuteriertem Chloroform mittels ¹⁹F-NMR bestimmt und betrug 63 %. Die Aufreinigung der Reaktionsmischung erfolgte über Säulenchromatographie (SiO₂, Hexane, Rf = 0.2) unter Verwendung einer Mikro-Säule.

¹H NMR (400 MHz, CDCl₃): δ = 7.45-7.29 (m, 10 H), 4.49 ppm (dp, J = 21.4, 7.9 Hz, 2H). ¹³C{¹H} NMR (101 MHz, CDCl₃): δ = 140.65, 140.42, 128.78, 128.63, 125.11, 125.03, 119.12, 115.78, 97.53, 95.66, 76.32 ppm (p, J = 12.0 Hz). ¹⁹F NMR (471 MHz, CDCl₃): δ = 83.13-81.87 (m), 70.95 (ddt, J = 147.9, 9.2, 8.7 Hz), -155.43 ppm (tp, J = 21.9, 11.8 Hz). HR-EI-MS *m*/*z* (calc.) = 326.0564 [*M*^{*·*+}]; *m*/*z* (found) = 326.0565 [*M·*⁺] C₁₄H₁₂F₆³²S.

### Addition von Schwefelhexafluorid an α-Methylstyrol

In einem Schlenkrohr wurden 5,9 mg (6,5 µL, 0,050 mmol, 1,00 eq.) α-Methylstyrol unter Argonatmosphäre in 1 mL absolutem MeCN gelöst. Anschließend wurden 0,7 mg (2,5 µmol, 5 Mol%) N-Phenylphenothiazin sowie 1,3 mg (5 µmol, 10 Mol%) Cu(acac)₂ zugegeben. Die Reaktionsmischung wurde drei Male unter Verwendung der gewöhnlichen Freeze-Pump-Thaw-Methode von Sauerstoff befreit. Anschließend wurde die Atmosphäre gegen Schwefelhexafluorid (1 bis 5 bar) getauscht. Das Reaktionsgemisch wurde bei 20°C für 21 h bei 365 nm unter Verwendung einer High-Power-LED belichtet/bestrahlt. Die Produktausbeute wurde nach Zugabe von deuteriertem Chloroform mittels ¹⁹F-NMR bestimmt und betrug 27 %. Die Aufreinigung der Reaktionsmischung erfolgte über Säulenchromatographie (SiO₂, n-Pentan, Rf = 0.3) unter Verwendung einer Pipettensäule.

Die strukturelle Charakterisierung des Produktes wurde wegen dessen hoher Flüchtigkeit in Gegenwart von n-Pentan durchgeführt.

¹H NMR (300 MHz, CDCl₃): d = 7.43-7.26 (m, 5H), 4.06 (ddp, J = 29.9, 22.7, 7.3 Hz, 2H), 1.86 ppm (d, J = 21.6 Hz, 3H). ¹³C{¹H} NMR (101 MHz, CDCl₃): d = 142.01, 128.88, 128.87, 128.48, 124.20, 124.11, 119.30, 115.96, 79.00, 78.02, 27.47 ppm (d, J = 17.5 Hz). ¹⁹F NMR (471 MHz, CDCl₃): d = 83.33-82.08 (m), 70.03 (ddt, J = 147.5, 9.2 Hz), -155.20-155.60 ppm (m). HR-EI-MS *m*/*z* (calc.) = 264.0407 [*M*^{*·*+}]; *m*/*z* (found) = 264.0408 [*M*^{*·*+}]C₉H₁₀F₆³²S.

Die vorliegende Erfindung stellt eine effiziente, nachhaltige und umweltfreundliche photoredoxkatalysierte Pentafluorsulfanylierungsmethode bereit. Die einzigartige Kombination aus photochemischem Abbau des starken Treibhausgases Schwefelhexafluorid unter Darstellung synthetisch wertvoller Verbindungen als pentafluorsulfanylierte Bausteine leistet einen wesentlichen Beitrag zur nachhaltigen Chemie. Die Zugänglichkeit derartiger Verbindungen war bislang durch die Verfügbarkeit und Toxizität der benötigten Reagenzien limitiert.

Das erfindungsgemäße Verfahren ermöglicht die Addition von Schwefelhexafluorid an ungesättigte Kohlenstoff-Kohlenstoff Doppelbindung Ohne durch eine bestimmte Theorie eingeschränkt zu sein, wird angenommen, dass es sich bei der Reaktion um einen konsekutiven Zweielektronenabsorptionsprozess handelt. In einem ersten Anregungsprozess wird der Photoredoxkatalysator (besonders bevorzugt N-Phenylphenothiazin) angeregt und ein erster Elektronentransfer auf SF₆ initiiert. Das entstehende Radikalkation des Katalysators wird erneut angeregt und oxidiert final das Substrat (Edukt) unter Rückbildung des Katalysators. Dieser Mechanismus entspricht dem Prinzip der Photoredoxkatalyse. Erfindungsgemäß können durch Erhöhung der Lebensdauer des vermutlich erzeugten Pentafluorsulfanylradikals (beispielsweise durch Gegenwart eines Radikalstabilisators im Reaktionsgemisch) eine besonders hohe Ausbeute an pentafluorsulfanyliertem Produkt sowie ein selektiver Reaktionsverlauf erhalten werden. Gemäß der vorliegenden Erfindung findet zum einen eine selektive Aktivierung durch Reduktion des Schwefelhexafluorids mithilfe von Licht sowie ein atomökonomischer Abbau von Schwefelhexafluorid unter Bildung des pentafluorsulfanylierten Produkts statt. Somit können pentafluorsulfanylierte Produkte, zu denen wertvolle und pharmakologisch potentiell wichtige Verbindungen zählen, effizient, nachhaltig und umweltschonend erhalten werden. Das erfindungsgemäße Verfahren kann für bestimmte Substrate durch die Anregung des Photoredoxkatalysators durch Licht die Addition von Schwefelhexafluorid an ungesättigte Kohlenstoff-Kohlenstoff-Bindungen mit hohen Ausbeuten erzielen. Zudem ist es möglich, durch das erfindungsgemäße Verfahren das umweltschädliche Treibhausgas SF₆ abzubauen.

## Patentansprüche

1. Verfahren zur photoredoxkatalytischen Addition von Schwefelhexafluorid an ein Edukt mit ungesättigter Kohlenstoff-Kohlenstoff-Bindung, umfassend die Schritte
(A) Bereitstellen eines Reaktionsgemisches, welches das Edukt, einen Photoredoxkatalysator sowie Schwefelhexafluorid, umfasst; und
(B) Bestrahlen des Reaktionsgemisches mit elektromagnetischer Strahlung, welche UV-Licht und/oder Licht aus dem sichtbaren Wellenlängenbereich ist, aus einer High-Power-LED als Lichtquelle,
wobei
der Photoredoxkatalysator ein Photoredoxkatalysator mit einer N-Phenylphenothiazin-Grundstruktur ist und
die ungesättigte Kohlenstoff-Kohlenstoff-Bindung eine Doppelbindung ist.

2. Verfahren nach Anspruch 1, wobei
der Photoredoxkatalysator eine Verbindung der folgenden Formel (1) umfasst 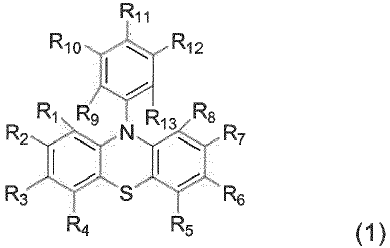 wobei die Reste R₁ bis R₁₃ unabhängig voneinander ein Wasserstoffatom, ein Deuteriumatom, ein Halogenatom, eine Cyanogruppe, eine Nitrogruppe, eine Trifluormethylgruppe, ein Fluoratom, eine substituierte oder unsubstituierte Kohlenwasserstoffgruppe, welche gegebenenfalls ein oder mehrere Heteroatome enthält, oder einen substituierten oder unsubstituierten aromatischen Rest darstellen.

3. Verfahren nach Anspruch 1 oder 2, wobei
der Photoredoxkatalysator N-Phenylphenothiazin ist.

4. Verfahren nach einem der Ansprüche 1 bis 3, wobei
das Edukt eine Verbindung der folgenden Formel (I) umfasst 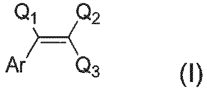 wobei
die Reste Q₁ bis Q₃ unabhängig voneinander ein Wasserstoffatom, ein Deuteriumatom, eine Cyanogruppe, eine Trifluormethylgruppe, eine substituierte oder unsubstituierte Kohlenwasserstoffgruppe, welche gegebenenfalls ein oder mehrere Heteroatome enthält, oder einen substituierten oder unsubstituierten aromatischen Rest darstellen und
Ar ein aromatischer Rest ist.

5. Verfahren nach einem der Ansprüche 1 bis 4, wobei
das Edukt eine Verbindung mit einer Styrol-Grundstruktur ist.

6. Verfahren nach einem der Ansprüche 1 bis 5, wobei
die elektromagnetische Strahlung Licht aus dem sichtbaren Wellenlängenbereich mit einer Wellenlänge von 400 bis 500 nm und/oder UV-A Licht ist.

7. Verfahren nach einem der Ansprüche 1 bis 6, wobei
das Reaktionsgemisch weiter einen Radikalstabilisator enthält.

8. Verfahren nach Anspruch 7, wobei
der Radikalstabilisator Kupferacetylacetonat ist.

## Claims

1. A method for the photoredox-catalytic addition of sulfur hexafluoride to a reactant with an unsaturated carbon-carbon bond, comprising the steps of:
(A) providing a reaction mixture comprising the reactant, a photoredox catalyst and sulfur hexafluoride; and
(B) irradiating the reaction mixture with electromagnetic radiation, which is UV light and/or light from the visible wavelength range, out of a high power LED as light source,
wherein
the photoredox catalyst is a photoredox catalyst with an N-phenylphenothiazine basic structure, and
the unsaturated carbon-carbon bond is a double bond.

2. The method according to claim 1, wherein
the photoredox catalyst comprises a compound of the following formula (1) 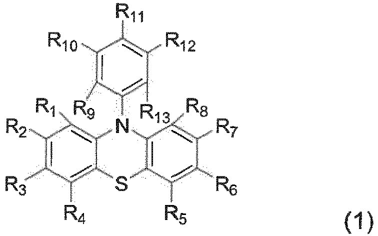 wherein R₁ to R₁₃ independently represent a hydrogen atom, a deuterium atom, a halogen atom, a cyano group, a nitro group, a trifluoromethyl group, a fluorine atom, a substituted or unsubstituted hydrocarbon group that optionally contains one or more heteroatoms, or a substituted or unsubstituted aromatic residue.

3. The method according to claim 1 or 2, wherein the photoredox catalyst is N-phenylphenothiazine.

4. The method according to one of claims 1 to 3, wherein the reactant comprises
a compound of the following formula (I) 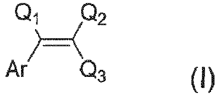 wherein
the residues Q₁ to Q₃ independently represent a hydrogen atom, a deuterium atom, a cyano group, a trifluoromethyl group, a substituted or unsubstituted hydrocarbon group that optionally contains one or more heteroatoms, a substituted or unsubstituted aromatic residue, and
Ar is an aromatic residue.

5. The method according to one of claims 1 to 4, wherein the reactant is a compound with a styrene basic structure.

6. The method according to one of claims 1 to 5, wherein the electromagnetic radiation is light from the visible wavelength range with a wavelength of 400 to 500 nm and/or UV-A light.

7. The method according to one of claims 1 to 6, wherein the reaction mixture further contains a radical stabilizer.

8. The method according to claim 7, wherein the radical stabilizer is copper acetylacetonate.

## Revendications

1. Procédé d'addition catalytique photoredox d'hexafluorure de soufre sur un réactif ayant une liaison carbone-carbone insaturée, comprenant les étapes de :
(A) fourniture d'un mélange réactionnel comprenant le réactif, un catalyseur photoredox et de l'hexafluorure de soufre ; et
(B) irradiation du mélange réactionnel avec un rayonnement électromagnétique, qui est de la lumière UV et/ou de la lumière de la plage de longueurs d'onde visibles, provenant d'une DEL à haute énergie comme source de lumière,
dans lequel
le catalyseur photoredox est un catalyseur photoredox ayant une structure à base de N-phénylphénothiazine ; et
la liaison carbone-carbone est une double liaison.

2. Procédé selon la revendication 1, dans lequel le catalyseur photoredox comprend un
composé ayant la formule suivante (1) 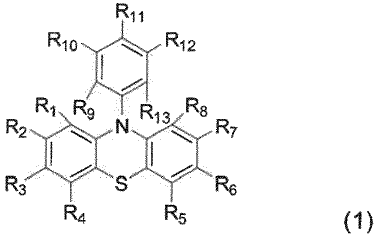 dans laquelle R₁ à R₁₃ représentent indépendamment, un atome d'hydrogène, un atome de deutérium, un atome d'halogène, un groupe cyano, un groupe nitro, un groupe trifluorométhyle, un atome de fluor, un groupe hydrocarboné substitué ou non substitué, qui contient le cas échéant, un ou plusieurs hétéroatomes, ou un résidu aromatique substitué ou non substitué.

3. Procédé selon la revendication 1 ou 2, dans lequel le catalyseur photoredox est la N-phénylphénothiazine.

4. Procédé selon l'une quelconque des revendications 1 à 3, dans lequel le réactif comprend un composé de la formule (I) suivante 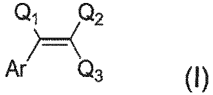 dans laquelle
les résidus Q₁ à Q₃ représentent indépendamment, un atome d'hydrogène, un atome de deutérium, un groupe cyano, un groupe trifluorométhyle, un groupe hydrocarboné substitué ou non substitué, qui contient le cas échéant, un ou plusieurs hétéroatomes, un résidu aromatique substitué ou non substitué, et
Ar est un résidu aromatique.

5. Procédé selon l'une quelconque des revendications 1 à 4, dans lequel le réactif est un composé ayant une structure à base de styrène.

6. Procédé selon l'une quelconque des revendications 1 à 5, dans lequel le rayonnement électromagnétique est de la lumière de la plage de longueurs d'onde visibles allant de 400 à 500 nm et/ou de la lumière UV-A.

7. Procédé selon l'une quelconque des revendications 1 à 6, dans lequel le mélange réactionnel contient en outre, un stabilisant radicalaire.

8. Procédé selon la revendication 7, dans lequel le stabilisant radicalaire est l'acétylacétonate de cuivre.
